(19) Europäisches Patentamt / European Patent Office / Office européen des brevets

(11) **EP 1 688 179 B1**

(12) **EUROPEAN PATENT SPECIFICATION**

(45) Date of publication and mention
of the grant of the patent:
**30.06.2010 Bulletin 2010/26**

(51) Int Cl.:
*B01J 19/00* (2006.01)   *B01L 3/00* (2006.01)
*G01N 27/22* (2006.01)   *G01N 27/00* (2006.01)
*G01N 29/02* (2006.01)   *G01N 33/543* (2006.01)
*G01N 33/68* (2006.01)   *G01N 33/557* (2006.01)
*C40B 30/04* (2006.01)

(21) Application number: **05257990.1**

(22) Date of filing: **22.12.2005**

(54) **Dissociation constant measuring apparatus**

Vorrichtung zur Dissoziationskonstantenmessung

Appareil de mesure de la constante de dissociation

(84) Designated Contracting States:
**DE FR GB SE**

(30) Priority: **02.02.2005 JP 2005026030**

(43) Date of publication of application:
**09.08.2006 Bulletin 2006/32**

(73) Proprietor: **Seiko Instruments Inc.**
**Chiba-shi, Chiba (JP)**

(72) Inventors:
• **Yoko Shinohara**
**c/o Seiko Instruments Inc**
**Chiba-shl, chiba (JP)**
• **Minao Yamamoto**
**c/o Seiko Instruments Inc**
**Mihama-ku2Chiba-shi, Chiba (JP)**
• **Masataka Shinogi**
**c/o Seiko Instruments Inc**
**Chiba-shi, Chiba (JP)**
• **Fumio Kimura**
**c/o Seiko Instruments Inc**
**Chiba (JP)**

• **Haruki Kato**
**c/o Seiko Instruments Inc**
**Mihama-ku**
**chiba-shi, Chiba (JP)**

(74) Representative: **Cloughley, Peter Andrew et al**
**Miller Sturt Kenyon**
**9 John Street**
**London WC1N 2ES (GB)**

(56) References cited:
**EP-A1- 1 403 641     DE-A1- 19 644 290**
**DE-A1- 19 706 486     US-A- 5 324 633**
**US-A- 5 674 742     US-A1- 2003 124 623**

• **ABDICHE Y N ET AL: "Probing the mechanism of drug/lipid membrane interactions using Biacore" ANALYTICAL BIOCHEMISTRY, ACADEMIC PRESS, SAN DIEGO, CA, US, vol. 328, no. 2, 15 May 2004 (2004-05-15), pages 233-243, XP004504054 ISSN: 0003-2697**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a dissociation constant measuring apparatus, and relates to, for example, those used for search for a compound that bonds with abnormal protein.

In recent years, analyses of the human genome (genetic information of the human) have been completed and clarification of a relation between abnormal protein generated by an abnormal genetic structure and diseases has been in progress.

Through the clarification of the relation, a developing method for new drugs is changing from an existing method relaying on experiences and intuitions of developers concerning drugs and compounds to a method of searching for a compound directly acting on abnormal protein to use the compound as a new drug. By adopting this method, it is expected that a period for development of a new drug, which requires nearly twenty years, is reduced to about five years in future.

For search for a compound as a candidate of a new drug, it is a general practice to use a dissociation constant, which is a constant representing a physical bonding force of abnormal protein and the compound as a candidate of a new drug, is used as a label.

As a measuring method for the dissociation constant, previously, a dissociation constant is calculated by using a compound obtained by bonding labeled substances such as an enzyme, a fluorescent material, and a radioactive isotope and, after bonding this compound and abnormal protein, measuring a quantity of the labeled substance to determine the bonded compound. However, at present, measurement performed by a micro-reactor without using the labeled substance attracts attention.

The micro-reactor is a device in which an lead-in path and a waste liquid path are formed on a chip constituted by semiconductor, glass, resin, or the like and a reaction tank is provided between the lead-in path and the waste liquid path. A sensor, to which abnormal protein is fixed in advance, is set in the reaction tank.

In the micro-reactor constituted in this way, when a sample solution containing a compound is poured from the lead-in path, the compound in the sample solution reacts with the abnormal protein set in the reaction tank in advance and a waste liquid after the reaction is discharged from the waste liquid path.

Note that a substance fixed in advance in the reaction tank is called ligand and a substance supplied as a solution is called analyte. Thus, these terms are used in the following description.

In the reaction tank, some of analytes in the sample solution bond with ligands to be fixed to the sensor. On the other hand, some of analytes fixed to the sensor separate from ligands to be discharged into the sample solution.

When the reaction of analytes and the ligands reach an equilibrium state (i.e., when a quantity of the analytes fixed to the sensor and a quantity the analytes separating from the sensor are equal), a quantity of the analytes fixed to the ligands is fixed. A dissociation constant is calculated from the number (molar concentrations) of a compound in which the analytes and the ligands have bonded, the number of the ligands that have not bonded with the analytes, and the number of the analytes that have not bonded with the ligands measured at this point. This dissociation constant is data necessary in search for a new drug.

As a technique concerning such a micro-reactor, there is a "micro-reactor" described below.

[Patent Document 1] WO 01/68257
This technique is a technique for forming micro-reactors such as a micro mixing chamber, a heating/dispersing unit, a reaction chamber, and a separating unit on a chip in parallel to one another to produce a large quantity of products.

As a technique for measuring bonding of ligands and analytes, there is an "immunoassay method" described below.

[Patent Document 2] Japanese Patent No. 2962031 This technique is a technique for detecting bonding of ligands and analytes using an acoustic sensor.

Conventionally, to calculate a dissociation constant, reaction of ligands and analytes is required to reach an equilibrium state, which takes a long time. Thus, there is a problem in that it takes time to obtain a dissociation constant. Since there are enormous combinations of ligands and analytes related to search, there is a problem in terms of cost in continuing an experiment until the reaction reaches equilibrium for all the combinations.

There is also a problem in that a large quantity of expensive samples are consumed by performing an experiment for a long time.

There is a request for making it possible to predict to some extent whether a combination is a promising combination at an initial stage of reaction. However, accurate prediction is difficult. A reason for this difficulty will be explained.

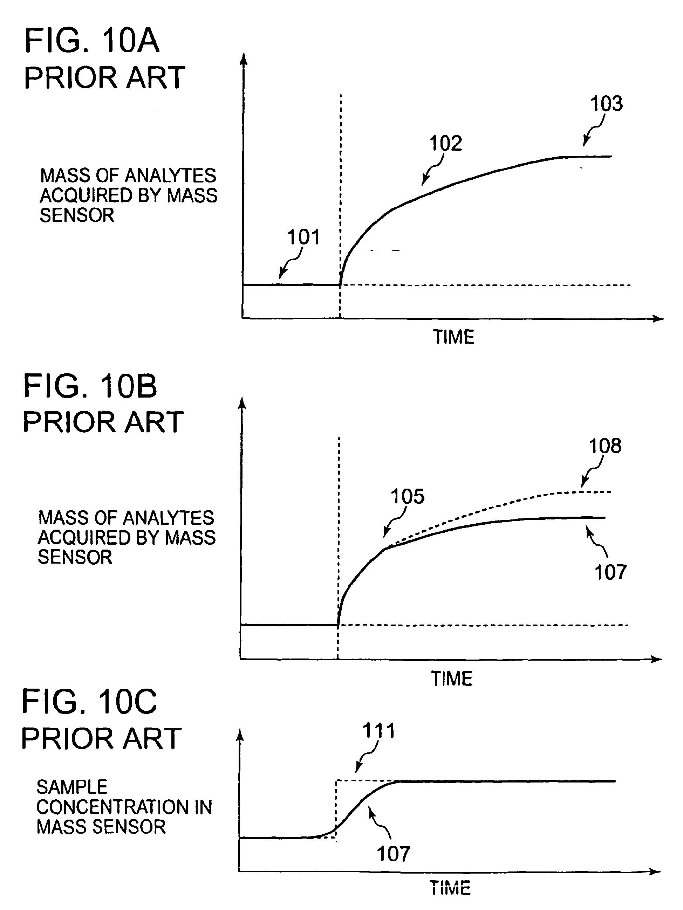

Fig. 10A is a graph representing a temporal change of a mass of analytes fixed to a sensor. An ordinate represents the mass of the analytes fixed to the sensor and an abscissa represents a time axis.

First, in a section 101, a buffer solution is supplied to a micro-reactor. Since analytes are not contained in the buffer solution, a mass of the analytes fixed to the sensor is zero. The buffer solution is fed in this way prior to a sample solution to prevent abnormal protein fixed to the sensor as ligands from being dried.

When solution supplied to the micro-reactor is switched from the buffer solution to the sample solution, a mass of the analytes fixed to the sensor gradually increases as indicated in a section 102. When the reaction of the ligands and the analytes reaches equilibrium, a mass of the analytes fixed to the sensor takes a fixed value as indicated in a section 103.

It is assumed that, for example, at a point 105 in Fig. 10B, a mass of the analytes after that is predicted as indicated by a curve 107 from a shape of a curve to that point. However, when measurement is actually continued, for example, a curve 108 is obtained. The mass significantly deviates from a predicted value.

As a major cause of such difficulty in the prediction, when the solution supplied to the sensor is switched from the buffer solution to the sample solution, since concentration of the sample gradually changes in the sensor, the concentration of the sample solution in the sensor is unknown at an initial stage of the reaction.

Fig. 10C is a graph showing a temporal change of sample concentration in the sensor. As indicated by a curve 110, the concentration of the sample gradually increases as time passes. This increase occurs because, after the solution supplied to the micro-reactor is switched from the buffer solution to the sample solution, both the buffer solution and the sample solution diffuse and mix in a lead-in path, heavy analytes concentrate in a high-flow rate area because of a flow rate distribution in a flow path, or the analytes adhere to a wall of the lead-in path leading to the sensor.

As indicated by a broken line 111, if the solution changes from the buffer solution to the sample solution alternatively at a certain point, a concentration of the sample in the sensor is known. However, actually, even if a change in the mass is detected by the sensor, since the course of a change in concentration of the sample to that point is unknown, it is difficult to predict concentration of the sample.

US 2003/124623 describes a microfluidic device for detecting analytes in a fluid using solid-phase affinity binding assays. The device comprises a microfluidic channel between a top wall and a substrate. Fluid flows into an inlet toward an outlet. A storage area contains a solid reagent. At least part of the wall of the substrate downstream of the reagent forms a detection area having a binding wall. This is preferably composed of a thin layer of gold and is suitable for use with a surface plasmon resonance detection process. In use, the channel is wetted with a first carrier fluid introduced into the inlet. This carrier fluid flows over the storage area, causing the matrix component of the reagent to dissolve within the carrier fluid. As the carrier fluid passes toward the outlet, the dissolved or suspended reagent diffuses within the carrier fluid, creating a reagent plume. Reagent molecules from this plume bind to the binding wall to form a concentration gradient of bound reagent.

In US 5,324,633 an apparatus is described for measuring the binding affinity of a receptor to a ligand. The apparatus includes a reactor system for synthesizing polymers on a substrate. The reactor system includes a body having a cavity on its top surface. The cavity has an array of ridges extending traverse to the plane of the paper. These ridges create turbulence for better mixing. A transparent substrate is mounted above the cavity. The substrate seals the cavity except for an inlet port and an outlet port. Fluid is pumped through the inlet port into the cavity via a pump. A mirror directs light from a light source onto selected parts of the substrate due to the use of a mask. Also, arrays of polymers are synthesized on the substrate. These arrays are separated by a channel block, which is attached to the substrate. Each of the arrays is subjected to a solution containing a selected receptor R. A first solution has a lower receptor concentration than a second solution. The receptor binds to polymers B and C, but hardly at all to polymer A. More binding occurs in the second solution, due to its higher receptor concentration.

In the paper "Probing the mechanism of drug/lipid membrane interactions using Biacore" by Y.N. Abdiche and D.G. Myszka, Analytical Biochemistry 328 (2004) pp. 233-243, a test is described for determining which of a series of drugs is able to bind to liposome surfaces. The test employs a surface plasmon resonance (SPR) biosensor. Five different lipids are used and suitably prepared. Liposomes are diluted in a running buffer and captured to saturation across isolated flow cells. Lipid titrations are then performed. The drug samples are injected across lipid and lipid-free surfaces. Association and dissociation phases are then monitored. After each binding cycle, the sensor surface is regenerated in order to remove the lipid previously under test. The sensor surface is coated with a new liposome solution for the next binding cycle.

A microfabricated reactor is the subject of US 5,674,742. This reactor is suitable for biochemical reactions, in particular DNA-based reactions, such as the polymerase chain reaction, which require thermal cycling, since the inherently small size of the instrument facilitates rapid cycle times. The instrument includes three reagent chambers containing reactants. A target DNA molecule is placed in the first chamber. The reactants in the three chambers are connected by respective channels, which are associated with respective pumps, to a reaction chamber. The reaction chamber has a Lambwave transducer and a heater, and is connected to a detection chamber via a further channel.

It is an object of the invention to make it possible to predict a dissociation constant at an initial stage of reaction.

In order to attain the object, the invention provides dissociation constant measuring apparatus, as set forth in claim 1.

In this first constitution, the mass acquiring means may be constituted to acquire a resonance frequency of a resonant member fixing the acquisition substance as mass information (a second constitution).

In the first constitution, the mass acquiring means may be constituted to acquire speed of an elastic wave propagating in the solution flowing over the acquisition substance as mass information (a third constitution).

In the first constitution, the second constitution, and the third constitution, the concentration acquiring means may acquire dielectric constant information of the solution after the sample is acquired as concentration information (a fourth constitution).

According to the invention, it is possible to predict a dissociation constant at an initial stage of reaction of ligands and analytes.

Embodiments of the invention will now be described by way of further example only and with reference to the accompanying drawings, in which:

Fig. 1 is a plan view schematically showing a structure of a micro-reactor in an embodiment of the invention;
Fig. 2 is a diagram for explaining a structure of a mass sensor;
Fig. 3A-3B are a diagram for explaining a structure of a concentration sensor;
Fig. 4 is a diagram schematically showing a constitution of a micro-reactor of a SAW type;
Fig. 5 is a diagram for explaining a logical expression used for prediction;
Fig. 6A-6B are a graph in which values sampled by a measuring apparatus are plotted;
Fig. 7 is a flowchart for explaining procedures taken by the measuring apparatus to predict a dissociation constant;
Fig. 8 is a continuation of the flowchart;
Fig. 9 is a block diagram showing an example of a hardware configuration of the measuring apparatus according to the embodiment; and
Fig. 10A-10C are a diagram for explaining a conventional example.

(Outline of Embodiment)

A concentration sensor for a sample is provided in the vicinity on a downstream side of a mass sensor that acquires analytes. Consequently, it is possible to learn concentration of a sample solution at the time when a mass is detected by the mass sensor.

When the concentration and the mass are substituted in a predetermined logical expression described below, it is possible to obtain a predicted value of a dissociation constant.

The concentration sensor is constituted by a capacitor including electrodes opposed to each other. Since a dielectric constant changes when analytes are present between the electrodes, it is possible to detect an analyte concentration by measuring an electric capacitance.

Since it is possible to reduce a size of the capacity by, for example, adopting a roller screen structure described later, the micro-reactor can be kept in small in size.

(Details of Embodiment)

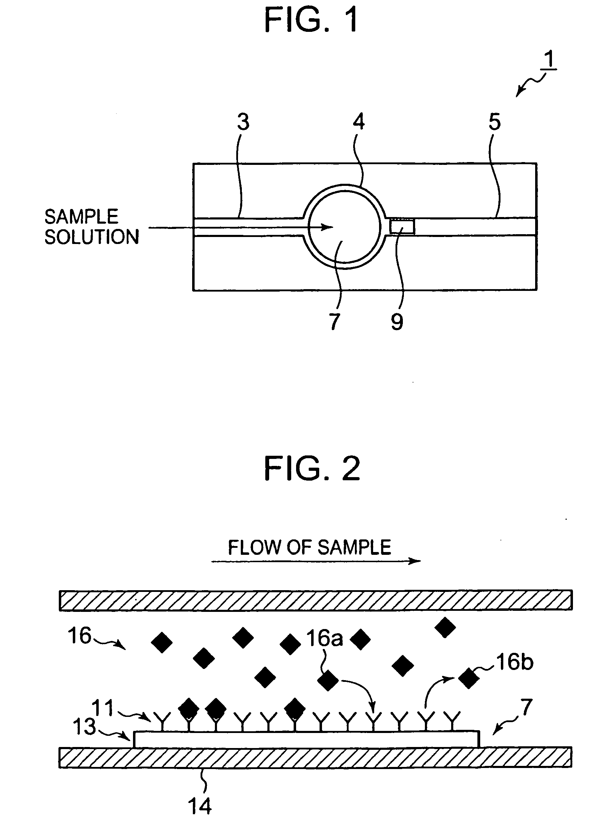

Fig. 1 is a plan view schematically showing a structure of a micro-reactor in this embodiment.

A micro-reactor 1 is constituted by a housing member made of, for example, glass. Various components are formed in this housing member.

As external dimensions of the micro-reactor 1, length (in a direction of an lead-in path 3) is about 50 to 100 [mm], width is about 10 to 20 [mm], and thickness is about several [mm].

The lead-in path 3, a reaction tank 4, a waste liquid path 5, and the like are formed in the micro-reactor 1. A mass sensor 7 is set in the reaction tank 4. A concentration sensor 9 is provided near the mass sensor 7 on the waste liquid path 5 side of the mass sensor 7.

Note that a valve for controlling flows of a buffer solution and a sample solution and wiring and the like constituting the mass sensor 7 and the concentration sensor 9 are not shown in the figure.

The lead-in path 3 is a flow path that leads a solution from the outside of the micro-reactor 1 into the reaction tank 4. A buffer solution and a sample solution containing analytes are lead to the reaction tank 4 through this pipe.

On the other hand, the waste liquid path 5 is a flow path that leads a solution discharged from the reaction tank 4 to the outside of the micro-reactor 1. The sample solution, which has finished reaction in the reaction tank 4, is discharged through this pipe.

In this embodiment, the solution is sucked from the side of the waste liquid path 5, whereby the solution is supplied from the lead-in path 3.

It is also possible to set a pump on the side of the lead-in path 3 to supply the solution to the micro-reactor 1. However, in this case, it is likely that analytes used in the previous experiment remain in the pump and the like and contaminate the present measurement to affect measurement accuracy (contamination). In this embodiment, such a problem is prevented by sucking the solution and supplying the solution to the micro-reactor 1.

The mass sensor 7 (mass acquiring means) has ligands fixed to the surface thereof and is constituted to be detachably attachable. A structure of the mass sensor 7 will be hereinafter explained using Fig. 2.

Fig. 2 shows a section of the reaction tank 4. The mass sensor 7 includes a gold thin film 13, ligands 11, and a quartz resonator 14.

The ligands 11 are fixed on a gold thin film 13 via a not-shown self-organizing film in advance. The quartz resonator 14 is provided on a lower surface of the gold thin film 13. The quartz resonator 14 forms a part of a Colpitts oscillator circuit and adapted to cause the quartz resonator 14 to resonate in a thickness slip oscillation mode and cause the gold thin film 13 to oscillate within a plane parallel to a flow of a sample. The gold thin film 13, the ligands 11, and the quartz resonator 14 constitute a resonating member.

Some of analytes 16 flowing over the mass sensor 7 bond with the ligands 11 (e.g., an analyte 16a) to be fixed to the mass sensor 7.

Even if the analytes are temporarily fixed to the mass sensor 7, some of the analytes are disconnected from the ligands and discharged into the sample solution again like an analyte 16b.

In this way, the ligands 11 constitute an acquisition substance that acquires a sample from the solution containing the sample (the analytes 16).

When the analytes are fixed to the mass sensor 7, since a mass of the mass sensor 7 increases, a resonance frequency of the mass sensor 7 shifts from fr to fr+$\Delta$f. It is possible to calculate a mass $\Delta$m of the analytes fixed to the mass sensor 7 by applying the following Expression (1) to this shift amount $\Delta$f . In the Expression, A is an area of the gold thin film 13, pq is density of quartz, and $\mu$q is a shearing stress of the quartz.

Note that the resonance frequency constitutes mass information representing a mass of the analytes fixed to the mass sensor 7.

$$\Delta f = 2fr^2 \Delta m / \{A(\rho q \mu q)^{1/2}\} \qquad (1)$$

A concentration sensor 9 (concentration acquiring means) will be explained.

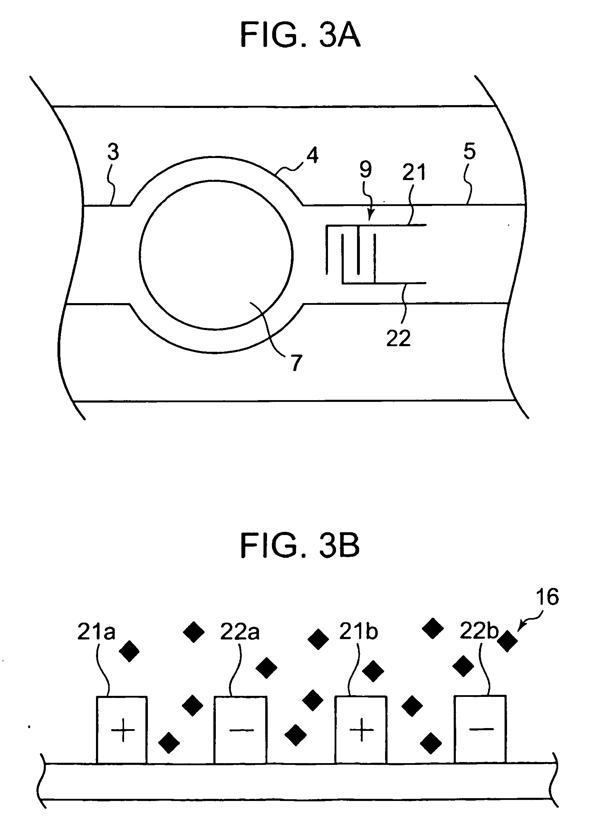

As shown in Fig. 3A, the concentration sensor 9 is constituted by a capacitor including an electrode 21 and an electrode 22. Fig. 3B shows sections of the electrodes 21 and 22.

The concentration sensor 9 is arranged coplanarly with and near the mass sensor 7. Since a concentration change is different in a height direction of a flow path, it is possible to predict a dissociation constant highly accurately by providing the concentration sensor 9 coplanarly with the mass sensor 7.

The electrodes 21 and 22 are arranged such that electrode sections formed in a convex shape on a bottom surface of the waste liquid path 5 are opposed to each other in the waste liquid path 5.

When the sample solution passes over the concentration sensor 9, the analytes in the sample solution come in and out of a space between the opposed electrodes 21 and 22 . A dielectric constant between the electrodes 21 and 22 changes according to a quantity of the analytes.

Since it is possible to associate a change in a dielectric constant and a change in an electric capacitance, it is possible to measure concentration of the sample solution according to an electric capacitance between the electrodes 21 and 22. The electric capacitance constitutes dielectric constant information and concentration information.

In this embodiment, a shape of the electrodes 21 and 22 is formed in a shape of a pair of roller screens to make it possible to obtain a large change in an electric capacitance with a small setting region area.

In other words, since the electrodes are arranged in a roller screen shape, as shown in Fig. 3B, it is possible to form three capacitors including electrodes 21a and 22a, electrodes 22a and 21b, and electrodes 21b and 22b, respectively.

Since it is possible to reduce a size of the concentration sensor 9 in this way, it is possible to prevent an increase in a size of the micro-reactor 1 due to the setting of the concentration sensor 9. This makes it possible to control an amount of use of a precious sample.

As described above, in the micro-reactor 1, since the concentration sensor 9 is provided in the vicinity on a downstream side of the mass sensor 7, it is possible to learn concentration of the sample solution at the time when $\Delta$m is detected by the mass sensor 7.

Note that, although $\Delta$m is calculated from transition of a resonance frequency of the quartz resonator 14 in the micro-reactor 1, it is also possible to measure $\Delta$m according to propagation speed of an elastic wave.

In general, a mass sensor of a type using a resonance frequency is called a QCM (Quartz Crystal Microbalance) and a mass sensor of a type using an elastic wave is called an SAW (Surface Acoustic Wave) sensor.

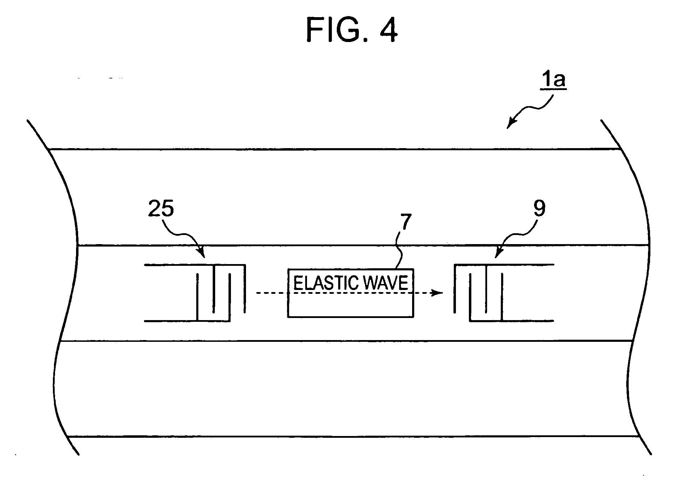

Fig. 4 is a diagram schematically showing a constitution of a micro-reactor 1a mounted with an SAW sensor.

An elastic wave generating electrode 25 is provided in the vicinity on an upstream side of the mass sensor 7.

A constitution of the elastic wave generating electrode 25 is the same as that of the concentration sensor 9. A pair of electrodes are arranged in a roller screen shape in the elastic wave generating electrode 25. When a voltage is applied to the elastic wave generating electrode 25, for example, a not-shown piezoelectric element expands and contracts to generate an elastic wave.

The concentration sensor 9 performs detection of an elastic wave and also performs detection of concentration of a sample solution at intervals of the detection of the elastic wave.

In this way, in the micro-reactor mounted with the SAW sensor, it is possible to cause the capacitor on the downstream side to function as both the concentration detecting means and the elastic wave detecting means.

Since a distance between the elastic wave generating electrode 25 and the concentration sensor 9 is known in advance, it is possible to calculate speed of an elastic wave passing over the mass sensor 7 according to a time difference between the time when the elastic wave generating electrode 25 generates an elastic wave and the time when the concentration sensor 9 detects the elastic wave.

It is known that, when analytes are captured by the mass sensor 7, transmission speed of an elastic wave decreases. It is possible to calculate a quantity of the analytes fixed to the mass sensor 7 from the speed of the elastic wave according to a predetermined logical expression.

Note that, in the case of the micro-reactor mounted with the SAW sensor, a width of the elastic wave generating electrode 25 and the concentration sensor 9 opposed to each other is sufficient as an area for detecting a mass (the mass sensor 7) (because, in areas other than the width of these electrodes, it is impossible to detect a mass even if analytes are bonded). It is unnecessary to form a reaction tank circular. Since it is possible to fix a width of the flow path, there is also an advantage that there is no pressure loss in this region and it is easy to perform feed control for the sample solution.

A prediction method for a dissociation constant using the micro-reactor 1 will be explained.

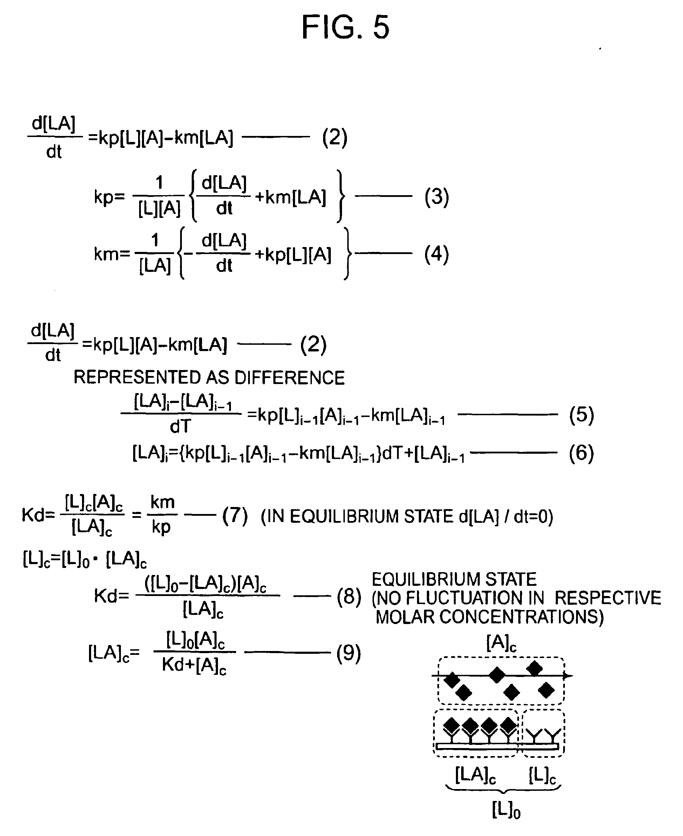

First, a logical expression used for prediction will be explained using Fig. 5. In the following explanation, ligands are represented by L and analytes are represented by A. A compound in which the ligands and the analytes have bonded is represented by LA. Note that [] indicates molar concentration of a substance written therein.

A relation represented by Expression (2) in Fig. 5 is established among LA, L, and A.

An amount of change in a very short time of the number of moles [LA] of the LA fixed to the mass sensor 7 by the bonding of the ligands and the analytes takes a value calculated by subtracting the number of moles (km[LA]), which decreases when temporarily captured analytes are released from the mass sensor 7, from the number of moles of the LA generated when the analytes are fixed to the mass sensor 7 (kp[L] [A]). In the equilibrium state, a first term and a second term on the right side are equal.

Note that, in the mass sensor 7, the number of moles of the LA fixed according to a reaction formula of $L+A \rightarrow LA$ is proportional to a number obtained by multiplying the number of moles [L] of the ligands that have not bonded with the analytes yet in the mass sensor 7 by the concentration (molar concentration) [A] of the analytes in the sample solution. A proportionality factor of the number of moles is set as kp.

The number of moles of the bonded analytes separating into the sample solution according to a reaction formula of $LA \rightarrow L+A$ in the LA formed in the mass sensor 7 is proportional to the number of moles of the LA formed in the mass sensor 7. A proportionality factor of the number of moles is set as km.

When Expression (2) is solved for kp and km, Expression (3) and Expression (4) are obtained, respectively.

On the other hand, when Expression (2) is represented by a difference, Expression (5) is obtained. Expression (5) is an expression obtained by rewriting Expression (2) using values obtained by sampling [LA], [L], and [A] at intervals of a very short time dT. A subscript i indicates how many times sampling is performed to obtain a sampling value. When Expression (5) is solved for $[LA]_i$, Expression (6) is obtained.

Incidentally, a dissociation constant Kd is represented by km/kp. In the equilibrium state, since d[LA]/dt=0, the dissociation constant Kd in an equilibrium state c is represented by Expression (7).

Since [L]c is represented as $[L]_c=[L]_0- [LA]c$ ($[L]_0$ is the number of moles of the ligands before the LA is formed), Expression (8) is derived. Expression (9) is derived from Expression (8).

Sampling of data will be explained.

A measuring apparatus described later acquires a resonance frequency of the mass sensor 7 and an electric capacitance of the concentration sensor 9 from the micro-reactor 1 at a sampling period dT and analyzes the resonance frequency and the electric capacitance to calculate the mass $\Delta m$ of the analytes fixed to the mass sensor 7 and the molar concentration [A] of the analytes in the sample solution at that point.

Consequently, it is possible to obtain $\Delta m$ and [A] at the interval of dT.

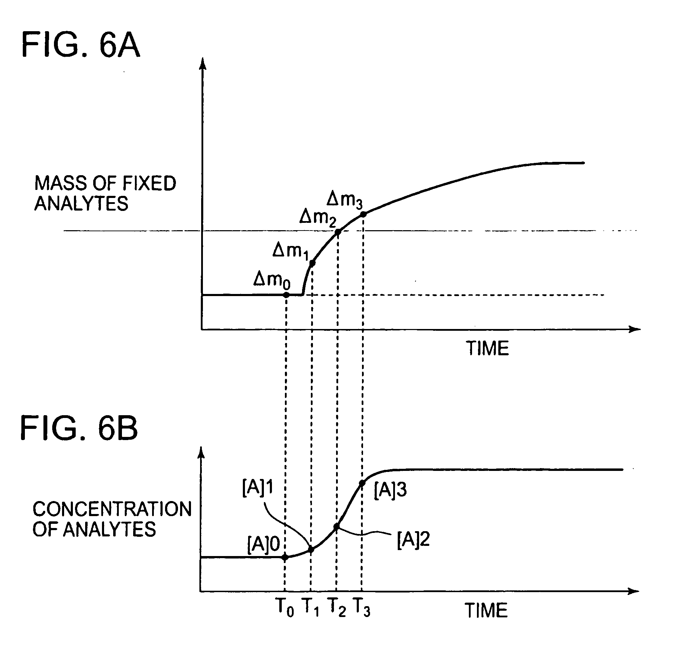

These values sampled by the measuring apparatus are plotted in Figs. 6(a) and 6(b).

Fig. 6A represents a mass of the analytes detected by the mass sensor 7. Fig. 6B represents a molar concentration of the analytes in the sample solution detected by the concentration sensor 9. Time axes of both the graphs are associated with each other.

As shown in Fig. 6A-6B, $\Delta m_0$ and $[A]_o$ are obtained at time To and $\Delta m_1$ and $[A]_1$ are obtained at time $T_1$ by the measuring apparatus. Similarly, after that, $\Delta m$ and [A] are detected at every dT.

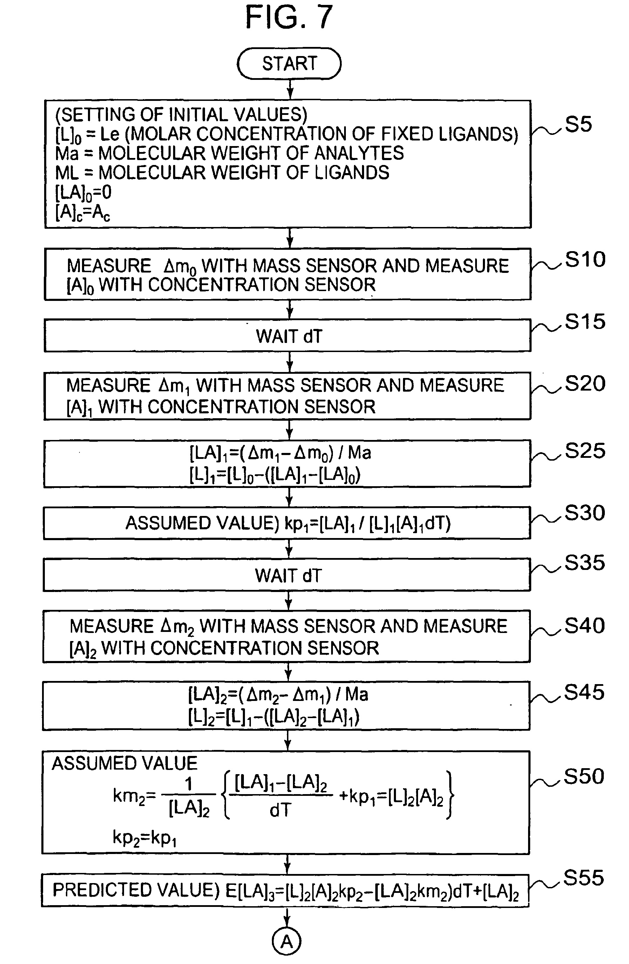

Procedures with which the measuring apparatus predicts a dissociation constant will be explained using a flowchart in Fig. 7.

First, the measuring apparatus sets initial values (step 5). In this initialization, the measuring apparatus sets the number of moles Le of the ligands fixed to the mass sensor 7 in advance to $[L]_0$, a molar concentration Ac of the analytes in a lead-in port of the micro-reactor to [A]c, and sets a molecular weight Ma of the analytes and a molecular weight ML of the ligands. A user inputs the values in the measuring apparatus to set the values. The measuring apparatus automatically sets [LA] to 0.

The measuring apparatus measures $\Delta m_0$ from an output of the mass sensor 7, measures $[A]_0$ from an output of the concentration sensor 9, and stores these values in a storage (step 10).

After waiting for the time dT (step 15), the measuring apparatus measures $\Delta m_1$ and $[A]_1$ and stores $\Delta m_1$ and $[A]_1$ (step 20).

The measuring apparatus calculates $[LA]_1$ and $[L]_1$ using these values stored (step 25).

$[LA]_1$ (the number of moles of the LA formed in the mass sensor 7) is obtained by dividing an increment ($\Delta m_1 - \Delta m_0$) of a mass detected by the mass sensor 7 by the molecular weight Ma of the analytes.

$[L]_1$ (the number of moles of the ligands that have not bonded with the analytes in the mass sensor 7) is obtained by subtracting the number of moles of the generated LA ($[LA]_1 - [LA]_0$) from the initial value $[L]_0$ of the ligands.

The measuring apparatus calculates an assumed value of $k_{P1}$ using $[LA]_1$, $[L]_1$, and [A] obtained and stores the assumed value (step 30). A formula for the calculation is as shown in the flowchart. This value is obtained by setting km[LA] to 0 in Expression (3). Since this is an initial stage where reaction of the ligands and the analytes has started, the number of the analytes released from the mass sensor 7 is set to 0.

After waiting for the time dT (step 35), the measuring apparatus measures $\Delta m_2$ and $[A]_2$ and stores $\Delta m_2$ and $[A]_2$ (step 40).

As in step 25, the measuring apparatus calculates $[LA]_2$ and $[L]_2$ and stores $[LA]_2$ and $[L]_2$ (step 45).

The measuring apparatus calculates assumed values of $km_2$ and $kp_2$ and stores the assumed values (step 50). Formulas for the calculation are as shown in the flowchart. A formula for $km_2$ uses Expression (5). Since it is considered that $kp_2$ has little difference from $kp_1$ during the time dT, $kp_2$ is assumed to be equal to $kp_1$.

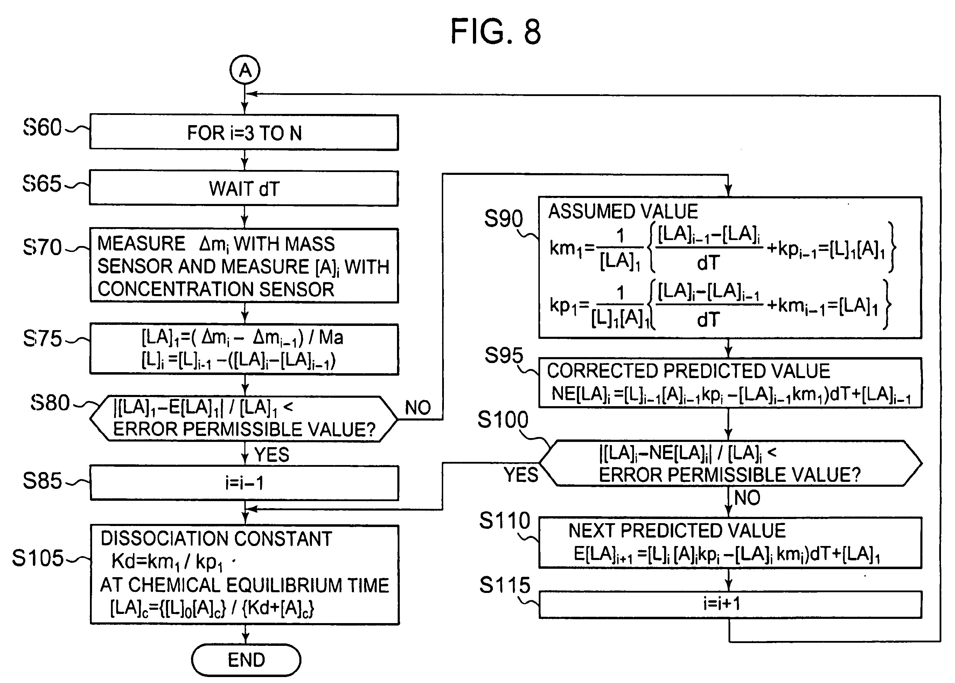

The measuring apparatus calculates a predicted value of $[LA]_3$ using $kp_2$, $km_2$, and the like (step 55). A formula for the calculation is as shown in the flowchart. This formula is obtained from Expression (6). The procedures continue to a flowchart in Fig. 8.

The measuring apparatus sets a counter i to 3 and performs the following loop processing until i reaches a predetermined integer N (step 60). Note that, in the following processing, the measuring apparatus calculates the dissociation constant Kd using an assumed value and evaluates the dissociation constant Kd. When the dissociation constant Kd enters a range of a permissible error, the measuring apparatus leaves the loop and outputs the dissociation constant Kd as a predicted value. The integer N is set to a sufficiently large value such that the loop processing continues until a predicted value of the dissociation constant Kd is calculated.

After waiting for the time dT (step 65), the measuring apparatus measures $\Delta m_i$ and $[A]_i$ and stores $\Delta m_i$ and $[A]_i$ (step 70).

As in step 25, the measuring apparatus calculates $[LA]_i$ and $[L]_i$ and stores $[LA]_i$ and $[L]_i$ in the storage (step 75).

The measuring apparatus performs evaluation of a prediction value of $[LA]_i$ (step 80). The measuring apparatus performs the evaluation by judging whether a value obtained by dividing a difference between $[LA]_i$ calculated in step 75 and the prediction value of $[LA]_i$ (the value calculated in step 55 when i=3 and the value calculated in step 110 in other cases) by $[LA]_i$ is smaller than an error permissible value set in advance.

When the difference between the calculated value and the predicted value of $[LA]_i$ is smaller than the error permissible value (step 80; Y), the measuring apparatus decrements i by 1 (step 85), calculates the dissociation constant Kd and $[LA]_c$ c at the time of chemical equilibrium and outputs the dissociation constant Kd and $[LA]_c$ (step 105), and ends the processing. Formulas for Kd and $[LA]_c$ are as shown in the flowchart. Note that the formula for $[LA]_c$ is according to Expression (9).

Note that Expression (9) is a relational expression derived from Expression (7) that is established when reactions of the ligands and the analytes are in an equilibrium state. Expression (9) is based on the idea that [A]c finally becomes the concentration Ac of the sample solution poured into the lead-in port of the micro-reactor.

On the other hand, when the difference between the calculated value and the predicted value of $[LA]_i$ is not smaller than the error permissible value (step 80; N), the measuring apparatus calculates assumed values of $km_i$ and $kp_i$ and stores the assumed values in the storage. Formulas for the calculation are as shown in the flowchart. Both the formulas use Expression (5) and are set to update assumed values using latest values $km_{i-1}$ and $kp_{i-1}$ presently stored.

The measuring apparatus calculates a corrected predicted value $NE[LA]_i$ of $[LA]_i$ in the same manner as step 55 using $km_i$ and $kp_i$ assumed in step 90 and stores the corrected predicted value in the storage (step 95). A formula for the calculation is as shown in the flowchart.

The measuring apparatus performs evaluation of the corrected predicted value $NE[LA]_i$ in the same manner as step 80 (step 100). An evaluation formula is as shown in the flowchart.

When a difference between the calculated value of $[LA]_i$ and the corrected predicted value $NE[LA]_i$ is smaller than the error permissible value set in advance (step 100; Y), in step 105 the measuring apparatus calculates the dissociation constant Kd and $[LA]_c$ at the time of chemical equilibrium and outputs the dissociation constant Kd and $[LA]_c$ (step 105).

When the difference between the calculated value of $[LA]_i$ and the corrected predicted value NE $[LA]_i$ is not smaller than the error permissible value set in advance (step 100; N), the measuring apparatus calculates the next predicted value E $[LA]_{i+1}$ in the same manner as step 55 (step 110). A formula for the calculation is as shown in the flowchart.

The measuring apparatus increments the counter i by one (step 115) and returns to step 60. Then, the measuring apparatus repeats the same processing.

In this way, the measuring apparatus includes the mass acquiring means that acquires, from the micro-reactor 1, mass information (a resonance frequency) representing a mass of a sample (analytes) in a solution acquired by an acquisition substance (ligands), the concentration acquiring means that acquires concentration information (an electric capacitance) representing concentration of the sample in the solution at the time when the sample is acquired by the acquisition substance, and the dissociation constant calculating means that calculates dissociation constants of the sample and the acquisition substance using the mass information and the concentration information acquired.

It is possible to constitute the measuring apparatus with the computer including the bonded substance quantity calculating means, the un-acquired quantity calculating means, and the dissociation constant calculating means. A measuring method by the measuring apparatus includes a bonded substance quantity calculating step of calculating, with the bonded substance quantity calculating means, a quantity of a bonded substance in which the sample and the acquisition substance are bonded ($[LA]_c$) using the mass information acquired by the mass acquiring means, an un-acquired quantity calculating step of calculating, with the un-acquired quantity calculating means, a quantity of an acquisition substance that has not acquired the sample ($[L]c=[L]0-[LA]c$) using the quantity of the bonded substance calculated, and a dissociation constant calculating step of calculating, with the dissociation constant calculating means, dissociation constants of the sample and the acquisition substance using the concentration information acquired in the concentration acquiring means ($[A]$), the quantity of the bonded substance calculated and the quantity of the acquisition substance calculated.

According to the procedures explained above, the measuring apparatus can predict a dissociation constant at an initial stage when the ligands and the analytes start to react with each other by sampling mass information and concentration information outputted from the micro-reactor 1. Therefore, a user can guess how promising combination of the ligands and the analytes is as a candidate of a new drug at the initial stage of the reaction of the ligands and the analytes.

Consequently, for example, it is possible to perform an operation such that, when the combination is expected to be a candidate of a new drug, an experiment is continued until bonding of the ligands and the analytes saturate and, when the combination is not expected to be a candidate of a new drug, the experiment is shifted to an experiment for the next combination.

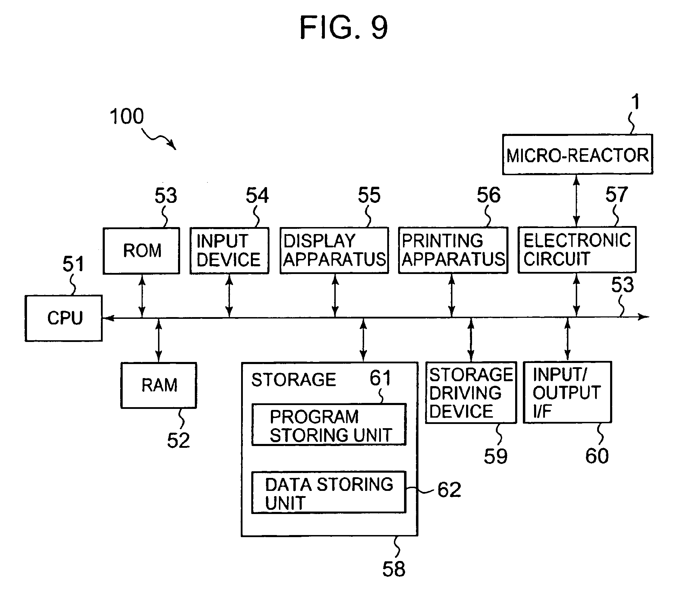

Fig. 9 is a block diagram showing an example of a hardware configuration of the measuring apparatus according to this embodiment.

A measuring apparatus 100 includes functional units such as a CPU (Central Processing Unit) 51, a RAM (Random Access Memory) 52, a ROM (Read Only Memory) 53, an input device 54, a display apparatus 55, a printing apparatus 56, an electronic circuit 57, a storage 58, a storage medium driving device 59, and an input/output I/F (interface) 60, which are connected by a bus line 50.

The CPU 51 is a central processing unit that performs various kinds of arithmetic processing, information processing, and control for respective components constituting the measuring apparatus 100 in accordance with predetermined programs.

The CPU 51 samples a resonance frequency and an electric capacitance outputted from the micro-reactor 1 via the electronic circuit 57 and calculates a mass of analytes fixed to the mass sensor 7 and concentration of a sampling solution using the values sampled. In addition, the CPU 51 calculates a predicted value of the dissociation constant Kd using the values.

The ROM 53 is a read-only storage having stored therein basic programs, data, and the like for operating the measuring apparatus 100.

The RAM 52 is a readable and writable storage that provides a working area for the CPU 51 to operate. When the CPU 51 performs information processing shown in Fig. 7, the RAM 52 stores values necessary for prediction such as $\Delta m0$, $\Delta m1$...

The input device 54 includes input devices such as a keyboard and a mouse. A user can input information necessary for using the measuring apparatus 100. It is possible to input the values described in step 5 in Fig. 7 from the input device 54.

The display apparatus 55 includes a display device that displays character information and image information. A menu screen and the like necessary for the user to use the measuring apparatus 100 are displayed on the display apparatus 55. It is also possible to display the predicted dissociation constant Kd on the display apparatus 55.

It is possible to constitute the display device with, for example, a liquid crystal display, a plasma display, and other display devices.

The electronic circuit 57 supplies power to the micro-reactor 1 and drives the micro-reactor 1. In addition, the electronic circuit 57 detects a resonance frequency of the mass sensor 7 and an electric capacitance of the concentration sensor 9 outputted from the micro-reactor 1.

Moreover, the electronic circuit 57 samples output data (analog data) of the micro-reactor 1 at the time interval dT, converts the output data into digital data, and provides the CPU 51 with the output data.

The CPU 51 calculates $\Delta$m and [A] from the sampled data. Consequently, $\Delta$m and [A] are obtained at the time interval dT.

The printing apparatus 56 is a printing apparatus such as a laser printer or an ink jet printer. The printing apparatus 56 can print experiment data.

The storage medium driving device 59 is a functional unit that drives an inserted detachable recording medium and performs reading and writing of data.

As a readable and writable recording medium, there are, for example, a flexible disk, a magneto-optical disk, a semiconductor storage, a magnetic tape, and a paper tape.

As a read-only recording medium, there are optical disks such as a CD-ROM.

The measuring apparatus 100 can perform installation and the like of a program from a storage medium inserted in the storage medium driving device 59. In addition, the measuring apparatus 100 can write experiment data in the storage medium.

The storage 58 is a large-capacity and readable and writable storage constituted by a hard disk or the like.

A program storing unit 61 storing programs and a data storing unit 62 storing data are formed in the storage 58.

Various programs such as an OS (Operating System) and a micro-reactor experiment program are stored in the program storing unit 61 to be executable by the CPU 51.

The OS is a program for causing the CPU 51 to show basic functions for operating the measuring apparatus 100 such as management of file input and output and control for the functional units.

The micro-reactor experiment program is a program for, for example, acquiring data, which is outputted from the micro-reactor 1, from the electronic circuit 57 and predicting a dissociation constant.

The CPU 51 can perform the information processing shown in Fig. 7 by executing this program.

Experiment data and the like obtained by the micro-reactor 1 are stored in the data storing unit 62.

As explained above, the micro-reactor in this embodiment is a micro-reactor in which, the reaction tank unit, the sample solution supply path that supplies a sample solution for analysis, the buffer solution supply path that supplies a buffer solution, and the waste liquid path for discharging a liquid to the outside are provided on a substrate. At least two pairs of electrodes of a roller screen shape provided on a piezoelectric substrate and the substance capturing means, which captures a specific substance, provided on the piezoelectric substrate and between the electrodes of a roller screen shape are provided in the reaction tank section. The micro-reactor can also include measuring means that measures an electric capacitance between at least one pair of electrodes of a roller screen shape, measuring means that measures an electric capacitance between the two pairs of electrodes of a roller screen shape, and electric means that generates an elastic wave between the two pairs of electrodes of a roller screen shape.

The embodiment of the invention has been explained. Advantages as described below are obtained through the embodiment.

(1) It is possible to provide the concentration sensor 9 in the micro-reactor 1.

(2) It is possible to realize a small concentration sensor having high detection sensitivity by constituting the concentration sensor 9 with electrodes of a roller screen shape.

(3) It is possible to detect, with the concentration sensor 9, concentration of a sample solution at the time when analytes are detected by the mass sensor 7.

(4) It is possible to predict a dissociation constant at high accuracy at an initial stage of reaction by sampling outputs of the mass sensor 7 and the concentration sensor 9 and substituting the outputs in a predetermined logical expression.

(5) It is possible to concentrate research resources on a promising candidate of a new drug using a predicted value of a dissociation constant.

(6) It is possible to prevent contamination by sucking a sample solution from the waste liquid path 5 side to supply the sample solution to the micro-reactor 1 and making the micro-reactor 1 disposable.

Note that, although the ligands are abnormal protein and the analytes are a compound of a candidate of a new drug in this embodiment, it is also possible that, conversely, the ligands are a candidate of a new drug and the analytes are abnormal protein.

The method of calculating a quantity of the analytes fixed to the mass sensor 7 is not limited to those using a resonance frequency and speed of an elastic wave. For example, it is also possible to use, for example, Surface Plasmon Resonance (SPR).

**Claims**

1. A dissociation constant measuring apparatus comprising:

   a micro-reactor, the micro-reactor comprising:

   a flow path (3, 5) through which, in use, a solution containing a sample (16) is fed, said flow path having an inlet end for the introduction of the solution;
   mass-information acquiring means (7) arranged to acquire mass information representing a mass of the sample in the solution, part of said sample being bonded to an acquisition substance (11) provided on the flow path; and
   concentration acquiring means (9) arranged to acquire concentration information representing a concentration of the sample in the solution at the time when the part of said sample is bonded to the acquisition substance, said concentration acquiring means being disposed in said flow path in a location which is more remote from said inlet end than said mass-information acquiring means, and said concentration acquiring means being a capacitor-type concentration sensor;
   whereby the mass information and concentration information can be acquired at an initial stage of reaction, at which the sample starts to react with the acquisition substance, and

   dissociation constant calculating means arranged to calculate a dissociation constant, Kd, of the sample and the acquisition substance using the mass information and the concentration information acquired by the micro-reactor at said initial stage of reaction, the dissociation constant calculating means being arranged to calculate the dissociation constant by the steps of:

   (a) determining values of parameters kp and km from the mass information and concentration information, parameter kp being a proportionality factor of the number of moles of the part of the sample (16) bonded with the acquisition substance (11) and parameter km being a proportionality factor of the number of moles of the part of the sample bonded to the acquisition substance, which subsequently separated back into the solution and were sensed by the concentration sensor; and
   (b) determining Kd as $Kd = km/kp$;
   wherein said step (a) comprises:

   (a1) setting initial values of the number of moles of the acquisition substance ($[L]0$, the molecular weight of the sample ($Ma$), the molecular weight of the acquisition substance ($ML$), the number of moles of the bonded sample, namely $[LA]_0 = 0$, and the number of moles of the sample in the inlet end of the flow path ($[A]c$;
   (a2) measuring mass ($\Delta m$) with the mass-information acquiring means (7) and a concentration of the sample ($[A]$) with the concentration acquiring means (9) at a first time t0, at a second time t1 and at a third time t2, where $t1=t0+dT$, $t2=t1+dT$ and $t3=t2+dT$, thereby yielding mass values $\Delta m0$, $\Delta m1$, $\Delta m2$, and sample concentration values $[A]_0$, $[A]_1$, $[A]_2$ ;
   (a3) from said initial values and the mass information ($\Delta m$) acquired at times t0 and t1, deriving (S25) a first value of the number of moles of the bonded sample, $[LA]_1$, and a first value of the number of moles of the unbonded acquisition substance, $[L]_1$; and from the mass information ($\Delta m$) acquired at times t1 and t2, deriving (S45) a second value of the number of moles of the bonded sample, $[LA]_2$, and a second value of the number of moles of the unbonded acquisition substance, $[L]_2$;
   (a4) deriving (S30) from $[LA]_1$, $[L]_1$ and $[A]_1$ an assumed first value of kp, namely $kp_1$; and deriving (S50) from $[LA]_1$, $[LA]_2$, ($[L]_2$) and $[A]_2$ an assumed value of km, namely $km_2$, wherein:

   $$km_2 = 1/[LA]_2(([LA]_1-[LA]_2)/dT + kp_1[L]_2[A]_2), \text{ and}$$
   $$kp_2 = kp_1;$$

   (a5) deriving (S55) from $km_2$ and $kp_2$ a first predicted value of the number of moles of the bonded sample, $E[LA]_3$, at an anticipated fourth time, t3, where $t3=t2+dT$, and wherein:

$$E[LA]_3 = ([L]_2[A]_2 kp_2 - [LA]_2 km_2)dT + [LA]_2;$$

(a6) at the fourth time t3, measuring (S70) mass ($\Delta m$) with the mass-information acquiring means (7) and a concentration of the sample ([A]) with the concentration acquiring means (9), thereby yielding a mass value, $\Delta m3$, and a sample concentration value, [A]3;

(a7) from the mass information $\Delta m3$ and $\Delta m2$, deriving (S75) a third value of the number of moles of the bonded sample, $[LA]_3$, and a third value of the number of moles of the unbonded acquisition substance $[L]_3$;

(a8) comparing (S80) $[LA]_3$ with $E[LA]_3$, and if the difference is less than a predetermined difference, proceeding to step (b);

(a9) if the difference is greater than the predetermined difference:

(a9-1) deriving (S90) an assumed third value of km and kp, namely $km_3$ and $kp_3$, wherein:

$$km_3 = 1/[LA]_3(([LA]_2 - [LA]_3)/dT + kp_2[L]_3[A]_3), \text{ and}$$
$$kp_3 = 1/[L]_3[A]_3(([LA]_3 - [LA]_2)/dT + km_2[LA]_3);$$

(a9-2) deriving (S95) a corrected predicted value of the number of moles of the bonded sample, $NE[LA]_3$, wherein:

$$NE[LA]_3 = ([L]_2[A]_2 kp_3 - [LA]_3)dT + LA]_2;$$

(a9-3) comparing (S100) $[LA]_3$ with $NE[LA]_3$, and, if the difference is less than the predetermined difference, proceeding to step (b), in which $Kd = km_3/kp_3$;

(a9-4) if the difference is greater than the predetermined difference, deriving (S110) from $km_3$ and $kp_3$ a second predicted value of the number of moles of the bonded sample, $E[LA]_4$, at an anticipated fifth time, t4, where t4=t3+dT, and wherein:

$$E[LA]_4 = ([L]_3[A]_3 kp_3 - [LA]_3 km_3)dT + [LA]_3; \text{ and}$$

(a9-5) iteratively repeating steps (a6)-(a9) for fifth and any required successive times, t4..., up to a maximum time, tN, until said difference is less than the predetermined difference.

2. The dissociation-constant measuring apparatus according to claim 1, wherein the mass-information acquiring means is arranged to determine; as mass information, a resonance frequency of a resonant member (14), to which is fixed the acquisition substance.

3. The dissociation-constant measuring apparatus according to claim 1, wherein the mass-information acquiring means is arranged to determine, as mass information, the speed of an elastic wave which, in use, propagates in the solution flowing over the acquisition substance.

4. The dissociation-constant measuring apparatus according to claim 1, wherein the mass-information acquiring means comprises a resonating member including a gold thin film (13), a plurality of ligands (11) and a quartz resonator (14), wherein:

the mass-information acquiring means is adapted so that the ligands are fixed on the gold thin film on one side thereof facing a flow of the sample (16), and the quartz resonator is provided on the other side thereof and forms part of a Colpitts oscillator circuit, the quartz resonator being adapted to resonate in a thickness slip oscillation mode, thereby causing the gold thin film to oscillate within a plane parallel to the flow of the sample.

5. The dissociation-constant measuring apparatus according to any one of the preceding claims, wherein the capacitor-type concentration sensor is arranged to acquire, as concentration information, dielectric constant information of the solution after the sample is acquired, said dielectric constant information being a value of a dielectric constant, which changes according to a quantity of analytes contained in said sample.

**Patentansprüche**

1.  Vorrichtung zum Messen einer Dissoziationskonstante, umfassend:

    einen Mikroreaktor, wobei der Mikroreaktor umfasst:

    einen Strömungspfad (3, 5), durch den, in Verwendung, eine Lösung, die eine Probe (16) enthält, zugeleitet wird, wobei der Strömungspfad ein Einlassende für das Einleiten der Lösung hat;
    ein Masseninformationsgewinnungsmittel (7), das zum Gewinnen von Masseninformationen angeordnet ist, die eine Masse der Probe in der Lösung darstellen, wobei ein Teil der Probe an eine Gewinnungssubstanz (11) gebunden ist, die an dem Strömungspfad bereitgestellt ist; und
    ein Konzentrationsgewinnungsmittel (9), das zum Gewinnen von Konzentrationsinformationen angeordnet ist, die eine Konzentration der Probe in der Lösung zu dem Zeitpunkt darstellen, wenn der Teil der Probe an die Gewinnungssubstanz gebunden ist, wobei das Konzentrationsgewinnungsmittel in dem Strömungspfad an einer Stelle angeordnet ist, die von dem Einlassende weiter entfernt ist als das Masseninformationsgewinnungsmittel, und das Konzentrationsgewinnungsmittel ein Konzentrationssensor der Kondensatorart ist;
    wodurch die Masseninformationen und Konzentrationsinformationen in einer Anfangsstufe einer Reaktion gewonnen werden können, in welcher die Probe beginnt, mit der Gewinnungssubstanz zu reagieren, und
    ein Dissoziationskonstante-Berechnungsmittel, das zur Berechnung einer Dissoziationskonstante Kd der Probe und der Gewinnungssubstanz unter Verwendung der Masseninformationen und der Konzentrationsinformationen angeordnet ist, die von dem Mikroreaktor in der Anfangsstufe einer Reaktion gewonnen werden, wobei das Dissoziationskonstante-Berechnungsmittel zur Berechnung der Dissoziationskonstante durch die folgenden Schritte angeordnet ist:

    (a) Bestimmen von Werten von Parametern kp und km aus den Masseninformationen und Konzentrationsinformationen, wobei der Parameter kp ein Proportionalitätsfaktor der Zahl von Molen des Teils der Probe (16) ist, der mit der Gewinnungssubstanz (11) gebunden ist, und der Parameter km ein Proportionalitätsfaktor der Zahl von Molen des Teils der Probe (16) ist, der an die Gewinnungssubstanz (11) gebunden ist, die anschließend wieder in die Lösung abgetrennt und von dem Konzentrationssensor erfasst wurden; und
    (b) Bestimmen von Kd als Kd = km/kp;

    wobei der Schritt (a) umfasst:

    (a1) Einstellen von Anfangswerten der Zahl von Molen der Gewinnungssubstanz ($[L]0$), des Molekulargewichts der Probe (Ma), des Molekulargewichts der Gewinnungssubstanz (ML), der Zahl von Molen der gebundenen Probe, nämlich $[LA]_0 = 0$, und der Zahl von Molen der Probe im Einlassende des Strömungspfades ($[A]c$);
    (a2) Messen der Masse ($\Delta m$) mit dem Masseninformationsgewinnungsmittel (7) und einer Konzentration der Probe ($[A]$) mit dem Konzentrationsgewinnungsmittel (9) zu einem ersten Zeitpunkt t0, einem zweiten Zeitpunkt t1 und einem dritten Zeitpunkt t2, wobei t1 = t0 + dT, t2 = t1 + dT und t3 = t2 + dT, wodurch Massenwerte $\Delta m0$, $\Delta m1$, $\Delta m2$ und Probenkonzentrationswerte $[A]_0$, $[A]_1$, $[A]_2$ erhalten werden;
    (a3) aus den Anfangswerten und den Masseninformationen ($\Delta m$), die zu Zeitpunkten t0 und t1 gewonnen wurden, Ableiten (S25) eines ersten Wertes der Zahl von Molen der gebundenen Probe, $[LA]_1$, und eines ersten Wertes der Zahl von Molen der ungebundenen Gewinnungssubstanz, $[L]_1$; und aus den Masseninformationen ($\Delta m$), die zu den Zeitpunkten t1 und t2 gewonnen wurden, Ableiten (S45) eines zweiten Wertes der Zahl von Molen der gebundenen Probe, $[LA]_2$, und eines zweiten Wertes der Zahl von Molen der ungebundenen Gewinnungssubstanz $[L]_2$;
    (a4) Ableiten (S30) aus $[LA]_1$, $[L]_1$ und $[A]_1$ eines angenommenen ersten Wertes kp, nämlich $kp_1$; und Ableiten (S50) aus $[LA]_1$, $[LA]_2$, $[L]_2$ und $[A]_2$ eines angenommen Wertes von km, nämlich $km_2$, wobei:

    $$km_2 = 1/[LA]_2(([LA]_1 - [LA]_2)dT + kp_1[L]_2[A]_2), \text{ und}$$
    $$kP_2 = kp_1;$$

    (a5) Ableiten (S55) aus $km_2$ und $kp_2$ eines ersten vorhergesagten Wertes der Zahl von Molen der gebundenen Probe $E[LA]_3$ zu einem antizipierten vierten Zeitpunkt t3, wobei t3 = t2 + dT, und wobei:

$$E[LA]_3 = ([L]_2[A]_2kP_2 - [LA]_2km_2)dT + [LA]_2;$$

(a6) zum vierten Zeitpunkt t3 Messen (S70) einer Masse ($\Delta$m) mit dem Masseninformationsgewinnungsmittel (7) und einer Konzentration der Probe ([A]) mit dem Konzentrationsgewinnungsmittel (9), wodurch ein Massenwert $\Delta$m3 und ein Probenkonzentrationswert [A]3 erhalten werden;

(a7) aus den Masseninformation $\Delta$m3 und $\Delta$m2 Ableiten (S75) eines dritten Wertes der Zahl von Molen der gebundenen Probe $[LA]_3$ und eines dritten Wertes der Zahl von Molen der ungebundenen Gewinnungssubstanz $[L]_3$;

(a8) Vergleichen von (S80) $[LA]_3$ mit E $[LA]_3$, und wenn die Differenz kleiner als eine vorbestimmte Differenz ist, Fortfahren mit Schritt (b);

(a9) wenn die Differenz größer als eine vorbestimmte Differenz ist:

(a9-1) Ableiten (S90) eines angenommenen dritten Wertes von km und kp, nämlich $km_3$ und $kp_3$, wobei:

$$km_3 = 1/[LA]_3(([LA]_2 - [LA]_3)dT + kp_2[L]_3[A]_3), \text{ und}$$
$$kP_3 = 1/[L]_3[A]_3(([LA]_3 - [LA]_2/dT + km_2[LA]_3);$$

(a9-2) Ableiten (S95) eines korrigierten vorhergesagten Wertes der Zahl von Molen der gebundenen Probe, $NE[LA]_3$, wobei:

$$NE[LA]_3 = ([L]_2[A]_2kp_3 - [LA]_2km_3) \, dT + [LA]_2;$$

(a9-3) Vergleichen (S100) von $[LA]_3$ mit $NE[LA]_3$ und wenn die Differenz geringer als die vorbestimmte Differenz ist, Fortfahren mit Schritt (b), in dem Kd = $km_3/kp_3$;

(a9-4) wenn die Differenz größer als die vorbestimmte Differenz ist, Ableiten (S110) aus $km_3$ und $kp_3$ eines zweiten vorhergesagten Wertes der Zahl von Molen der gebundenen Probe, $E[LA]_4$, zu einem antizipierten fünften Zeitpunkt, t4, wobei t4 = t3 + dT, und wobei:

$$E[LA]_4 = ([L]_3[A]_3kp_3 - [LA]_3km_3)dT + [LA]_3; \text{ und}$$

(a9-5) iteratives Wiederholen der Schritte (a6 bis a9) für fünfte und alle erforderlichen folgenden Zeitpunkte, t4..., bis zu einem maximalen Zeitpunkt tN, bis die Differenz geringer als die vorbestimmte Differenz ist.

2. Vorrichtung zum Messen einer Dissoziationskonstante nach Anspruch 1, wobei das Masseninformationsgewinnungsmittel angeordnet ist zum Bestimmen: als Masseninformationen, einer Resonanzfrequenz eines resonanten Elements (14), an dem die Gewinnungssubstanz fixiert ist.

3. Vorrichtung zum Messen einer Dissoziationskonstante nach Anspruch 1, wobei das Masseninformationsgewinnungsmittel angeordnet ist zum Bestimmen: als Masseninformationen, der Geschwindigkeit einer elastischen Welle, die sich, in Verwendung, in der Lösung fortpflanzt, die über die Gewinnungssubstanz strömt.

4. Vorrichtung zum Messen einer Dissoziationskonstante nach Anspruch 1, wobei das Masseninformationsgewinnungsmittel ein resonierendes Element umfasst, das einen Golddünnfilm (13), mehrere Liganden (11) und einen Quarzresonator (14) enthält, wobei:

das Masseninformationsgewinnungsmittel dazu ausgebildet ist, dass die Liganden an dem Golddünnfilm an einer Seite befestigt sind, die einem Fluss der Probe (16) gegenüber liegt, und der Quarzresonator an seiner anderen Seite bereitgestellt ist und einen Teil einer Colpitts-Oszillatorschaltung bildet, wobei der Quarzresonator dazu ausgebildet ist, in einem Dickengleit-Oszillationsmodus zu resonieren, wodurch der Golddünnfilm innerhalb einer Ebene, die parallel zu dem Strom der Probe liegt, in Schwingung versetzt wird.

5. Vorrichtung zum Messen einer Dissoziationskonstante nach einem der vorangehenden Ansprüche, wobei der Konzentrationssensor vom Kondensatortyp so angeordnet ist, dass er als Konzentrationsinformationen Informationen zur dielektrischen Konstante der Lösung gewinnt, nachdem die Probe gewonnen wurde, wobei die Informationen zur dielektrischen Konstante ein Wert einer dielektrischen Konstante sind, der sich entsprechend einer Menge an Analyten ändert, die in der Probe enthalten ist.

**Revendications**

1. Appareil de mesure de constante de dissociation comprenant :

   un microréacteur, le microréacteur comprenant :

   un canal (3, 5) grâce auquel, lors de l'utilisation, une solution contenant un échantillon (16) est acheminée, ledit canal ayant une extrémité d'entrée pour faire entrer la solution ;
   un moyen d'acquisition d'informations sur la masse (7) étudié pour acquérir de l'information sur la masse représentant une masse de l'échantillon dans la solution, une partie dudit échantillon étant en liaison avec une substance d'acquisition (11) fournie dans le canal ; et
   un moyen d'acquisition de la concentration (9) étudié pour acquérir une information sur la concentration représentant une concentration de l'échantillon dans la solution au moment où la partie dudit échantillon est liée à la substance d'acquisition, ledit moyen d'acquisition de la concentration étant disposé dans ledit canal dans un endroit plus éloigné de ladite extrémité d'entrée que ledit moyen d'acquisition d'informations sur la masse, et ledit moyen d'acquisition de la concentration étant un capteur de la concentration de type capacitif ;
   moyennant quoi l'information sur la masse et l'information sur la concentration peuvent être acquises lors d'une étape initiale de réaction, au cours de laquelle l'échantillon commence à réagir avec la substance d'acquisition, et
   le moyen de calcul de constante de dissociation étant étudié pour calculer une constante de dissociation Kd de l'échantillon et de la substance d'acquisition en utilisant l'information sur la masse et l'information sur la concentration acquises par le microréacteur au cours de ladite étape initiale de réaction, le moyen de calcul de constante de dissociation étant étudié pour calculer la constante de dissociation grâce aux étapes suivantes :

   a) détermination des valeurs des paramètres kp et km d'après l'information sur la masse et l'information sur la concentration, le paramètre kp étant un coefficient de proportionnalité du nombre de moles de la partie de l'échantillon (16) en liaison avec la substance d'acquisition (11) et le paramètre km étant un coefficient de proportionnalité du nombre de moles de la partie de l'échantillon en liaison avec la substance d'acquisition, lesquelles se sont ensuite séparées pour revenir dans la solution et ont été détectées par le capteur de la concentration ; et
   b) détermination de Kd avec Kd = km/kp ;

   ladite étape a) comprenant :

   a1) fixation de valeurs initiales du nombre de moles de la substance d'acquisition ($[L]0$), du poids moléculaire de l'échantillon ($Ma$), du poids moléculaire de la substance d'acquisition ($ML$), du nombre de moles de l'échantillon lié, à savoir $[LA]_0=0$, et du nombre de moles de l'échantillon dans l'extrémité d'entrée du canal ($[A]c$);
   a2) mesure de la masse ($\Delta m$) avec le moyen d'acquisition d'informations sur la masse (7) et d'une concentration de l'échantillon ($[A]$) avec le moyen d'acquisition de la concentration (9) à un premier moment t0, à un deuxième moment t1 et à un troisième moment t2, avec t1=t0+dT, t2=t1+dT et t3=t2+dT, en obtenant ainsi des valeurs de masse $\Delta m0$, $\Delta m1$, $\Delta m2$ et des valeurs de concentration de l'échantillon $[A]_0$, $[A]_1$, $[A]_2$ ;
   a3) à partir desdites valeurs initiales et de l'information sur la masse ($\Delta m$) acquise aux moments t0 et t1, en déduire (S25) une première valeur du nombre de moles de l'échantillon lié $[LA]_1$, et une première valeur du nombre de moles de la substance d'acquisition non liée $[L]_1$ ; et à partir de l'information sur la masse ($\Delta m$) acquise aux moments t1 et t2, en déduire (S45) une deuxième valeur du nombre de moles de l'échantillon lié $[LA]_2$, et une deuxième valeur du nombre de moles de la substance d'acquisition non liée, $[L]_2$ ;
   a4) obtenir (S30) à partir de $[LA]_1$, $[L]_1$ et $[A]_1$, une première valeur implicite de kp, à savoir $kp_1$ ; et obtenir (S50) à partir de $[LA]_1$, $[LA]_2$, $[L]_2$ et $[A]_2$, une valeur implicite de km, à savoir $km_2$, avec :

   $$km_2 = 1/[LA]_2(([LA]_1- [LA]_2/dT + kp_1[L]_2[A]_2), \text{ et}$$
   $$kp_2=kp_1 ;$$

   a5) obtenir (S55) à partir de $km_2$ et $kp_2$, une première valeur théorique du nombre de moles de l'échan-

tillon lié $E[LA]_3$ à un quatrième moment anticipé $t_3$, avec $t_3 = t_2 + dT$, et avec :

$$E[LA]_3 = ([L]_2[A]_2 kp_2 - [LA]_2 km_2)dT + [LA]_2;$$

a6) au quatrième moment t3, mesure (S70) de la masse ($\Delta m$) avec le moyen d'acquisition d'informations sur la masse (7) et d'une concentration de l'échantillon ([A]) avec le moyen d'acquisition de la concentration (9), en obtenant ainsi une valeur de la masse $\Delta m3$ et une valeur de concentration de l'échantillon [A]3 ;

a7) à partir de l'information sur la masse $\Delta m3$ et $\Delta m2$, en déduire (S75) une troisième valeur du nombre de moles de l'échantillon lié $[LA]_3$ et une troisième valeur du nombre de moles de la substance d'acquisition non liée $[L]_3$ ;

a8) comparer (S80) $[LA]_3$ avec $E[LA]_3$, et si la différence est inférieure à une différence prédéterminée, passer à l'étape b) ;

a9) si la différence est supérieure à la différence prédéterminée :

(a9-1) obtenir (S90) une troisième valeur implicite de km et kp, à savoir $km_3$ et $kp_3$, avec :

$$km_3 = 1/[LA]_3(([LA]_2 - [LA]_3/dT + kp_2[L]_3[A]_3),\ et$$
$$kp_3 = 1/[L]_3[A]_3(([LA]_3 - [LA]_2/dT + km_2[LA]_3)\ ;$$

(a9-2) obtenir (S95) une valeur implicite corrigée du nombre de moles de l'échantillon lié, $NE[LA]_3$, avec

$$NE[LA]_3 = ([L]_2[A]_2 kp_3 - [LA]_2 km_3)dT + [LA]_2)\ ;$$

(a9-3) comparer (S100)$[LA]_3$ avec $NE[LA]_3$, et si la différence est inférieure à la différence prédéterminée, passer à l'étape b), où $Kd = km_3/kp_3$ ;

(a9-4) si la différence est supérieure à la différence prédéterminée, obtenir (S110), à partir de $km_3$ et $kp_3$, une deuxième valeur théorique du nombre de moles de l'échantillon lié $E[LA]_4$ à un cinquième moment anticipé t4, où t4 = t3 + dT, et avec :

$$E[LA]_4 = ([L]_3[A]_3 kp_3 - [LA]_3 km_3)dT + [LA]_3)\ ;\ et$$

(a9-5) répéter de manière itérative les étapes (a6)-(a9) pour la cinquième fois et les fois suivantes selon le besoin, de t4... jusqu'à un nombre de fois maximal tN, jusqu'à ce que ladite différence soit inférieure à la différence prédéterminée.

2. Appareil de mesure de constante de dissociation selon la revendication 1, le moyen d'acquisition d'informations sur la masse étant étudié pour déterminer, en guise d'information sur la masse, une fréquence de résonance d'un élément résonant (14) sur lequel la substance d'acquisition est fixée.

3. Appareil de mesure de constante de dissociation selon la revendication 1, le moyen d'acquisition d'informations sur la masse étant étudié pour déterminer, en guise d'information sur la masse, la vitesse d'une onde élastique laquelle, lors de l'utilisation, se propage dans la solution s'écoulant sur la substance d'acquisition.

4. Appareil de mesure de constante de dissociation selon la revendication 1, le moyen d'acquisition d'informations sur la masse comprenant un élément résonateur comprenant une couche mince en or (13), une pluralité de ligands (11) et un résonateur à quartz (14) ;

le moyen d'acquisition d'informations sur la masse étant adapté de manière à ce que les ligands soient fixés sur la couche mince en or sur un côté de celle-ci en vis-à-vis d'un flux de l'échantillon (16), et le résonateur à quartz étant fourni sur l'autre côté de celle-ci et faisant partie d'un circuit oscillateur de Colpitts, le résonateur à quartz étant adapté pour résonner en mode d'oscillation à glissement dans le sens de l'épaisseur, en faisant de la sorte osciller la couche mince en or dans un plan parallèle au flux de l'échantillon.

5. Appareil de mesure de constante de dissociation selon l'une quelconque des revendications précédentes, le capteur de la concentration de type capacitif étant étudié pour acquérir, en guise d'information sur la concentration, une information sur la constante diélectrique de la solution une fois l'échantillon acquis, ladite information sur la constante diélectrique étant une valeur d'une constante diélectrique laquelle varie en fonction d'une quantité d'analytes se trouvant dans ledit échantillon.

# FIG. 1

1

SAMPLE
SOLUTION

3    4    5

7    9

# FIG. 2

FLOW OF SAMPLE

16    16a    16b

11
13
14    7

# FIG. 3A

# FIG. 3B

# FIG. 4

<u>1a</u>

25

7

9

ELASTIC WAVE

# FIG. 5

$$\frac{d[LA]}{dt} = kp[L][A] - km[LA] \quad\text{——— (2)}$$

$$kp = \frac{1}{[L][A]}\left\{\frac{d[LA]}{dt} + km[LA]\right\} \quad\text{——— (3)}$$

$$km = \frac{1}{[LA]}\left\{-\frac{d[LA]}{dt} + kp[L][A]\right\} \quad\text{——— (4)}$$

$$\frac{d[LA]}{dt} = kp[L][A] - km[LA] \quad\text{——— (2)}$$

REPRESENTED AS DIFFERENCE

$$\frac{[LA]_i - [LA]_{i-1}}{dT} = kp[L]_{i-1}[A]_{i-1} - km[LA]_{i-1} \quad\text{——— (5)}$$

$$[LA]_i = \{kp[L]_{i-1}[A]_{i-1} - km[LA]_{i-1}\}dT + [LA]_{i-1} \quad\text{——— (6)}$$

$$Kd = \frac{[L]_c[A]_c}{[LA]_c} = \frac{km}{kp} \quad\text{——— (7)} \quad \text{(IN EQUILIBRIUM STATE } d[LA]/dt=0\text{)}$$

$[L]_c = [L]_0 \cdot [LA]_c$

$$Kd = \frac{([L]_0 - [LA]_c)[A]_c}{[LA]_c} \quad\text{——— (8)}$$

EQUILIBRIUM STATE
(NO FLUCTUATION IN RESPECTIVE
MOLAR CONCENTRATIONS)

$$[LA]_c = \frac{[L]_0[A]_c}{Kd + [A]_c} \quad\text{——— (9)}$$

# FIG. 6A

# FIG. 6B

EP 1 688 179 B1

# FIG. 7

START

(SETTING OF INITIAL VALUES)
$[L]_0$ = Le (MOLAR CONCENTRATION OF FIXED LIGANDS)
Ma = MOLECULAR WEIGHT OF ANALYTES
ML = MOLECULAR WEIGHT OF LIGANDS
$[LA]_0=0$
$[A]_c=A_c$ — S5

MEASURE $\Delta m_0$ WITH MASS SENSOR AND MEASURE $[A]_0$ WITH CONCENTRATION SENSOR — S10

WAIT dT — S15

MEASURE $\Delta m_1$ WITH MASS SENSOR AND MEASURE $[A]_1$ WITH CONCENTRATION SENSOR — S20

$[LA]_1=(\Delta m_1 - \Delta m_0) / Ma$
$[L]_1=[L]_0-([LA]_1-[LA]_0)$ — S25

ASSUMED VALUE) $kp_1=[LA]_1 / [L]_1[A]_1 dT)$ — S30

WAIT dT — S35

MEASURE $\Delta m_2$ WITH MASS SENSOR AND MEASURE $[A]_2$ WITH CONCENTRATION SENSOR — S40

$[LA]_2=(\Delta m_2 - \Delta m_1) / Ma$
$[L]_2=[L]_1-([LA]_2-[LA]_1)$ — S45

ASSUMED VALUE
$km_2= \dfrac{1}{[LA]_2} \left\{ \dfrac{[LA]_1-[LA]_2}{dT} +kp_1=[L]_2[A]_2 \right\}$
$kp_2=kp_1$ — S50

PREDICTED VALUE) $E[LA]_3=[L]_2[A]_2kp_2-[LA]_2km_2)dT+[LA]_2$ — S55

Ⓐ

22

# FIG. 8

(A)

**S60** — FOR i=3 TO N

**S65** — WAIT dT

**S70** — MEASURE $\Delta m_i$ WITH MASS SENSOR AND MEASURE $[A]_i$ WITH CONCENTRATION SENSOR

**S75** — $[LA]_1 = (\Delta m_i - \Delta m_{i-1}) / Ma$
$[L]_i = [L]_{i-1} - ([LA]_i - [LA]_{i-1})$

**S80** — $|[LA]_1 - E[LA]_1| / [LA]_1 <$ ERROR PERMISSIBLE VALUE? — NO

YES

**S85** — $i = i - 1$

**S105** — DISSOCIATION CONSTANT
$Kd = km_1 / kp_1 \cdot$
AT CHEMICAL EQUILIBRIUM TIME
$[LA]_c = \{[L]_0 [A]_c\} / \{Kd + [A]_c\}$

END

**S90** — ASSUMED VALUE
$km_1 = \dfrac{1}{[LA]_1}\left\{\dfrac{[LA]_{i-1} - [LA]_i}{dT} + kp_{i-1} = [L]_1[A]_1\right\}$

$kp_1 = \dfrac{1}{[L]_1[A]_1}\left\{\dfrac{[LA]_i - [LA]_{i-1}}{dT} + km_{i-1} = [LA]_1\right\}$

**S95** — CORRECTED PREDICTED VALUE
$NE[LA]_i = [L]_{i-1}[A]_{i-1} kp_i - [LA]_{i-1} km_1)dT + [LA]_{i-1}$

**S100** — $|[LA]_i - NE[LA]_i| / [LA]_i <$ ERROR PERMISSIBLE VALUE? — YES

NO

**S110** — NEXT PREDICTED VALUE
$E[LA]_{i+1} = [L]_i [A]_i kp_i - [LA]_i km_i)dT + [LA]_1$

**S115** — $i = i + 1$

EP 1 688 179 B1

# FIG. 9

# FIG. 10A
## PRIOR ART

MASS OF ANALYTES
ACQUIRED BY MASS
SENSOR

TIME

# FIG. 10B
## PRIOR ART

MASS OF ANALYTES
ACQUIRED BY MASS
SENSOR

TIME

# FIG. 10C
## PRIOR ART

SAMPLE
CONCENTRATION IN
MASS SENSOR

TIME

25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0168257 A
- JP 2962031 B
- US 2003124623 A
- US 5324633 A
- US 5674742 A


**Non-patent literature cited in the description**

- **Y.N. Abdiche ; D.G. Myszka.** Probing the mechanism of drug/lipid membrane interactions using Biacore. *Analytical Biochemistry,* 2004, vol. 328, 233-243